# EUROPÄISCHE PATENTANMELDUNG

(11) **EP 0 747 015 A1**
(43) Veröffentlichungstag der Anmeldung: **11.12.1996**
(21) Anmeldenummer: 96108553.7
(22) Anmeldetag: 29.05.1996
(51) Int. Cl.: A61B 17/16, A61F 2/46

(54) **Instrumentarium zur Markraumvorbereitung**

(30) Priorität: 09.06.1995 DE 19521053
(71) Anmelder: MERCK PATENT GmbH, 64293 Darmstadt (DE)
(72) Erfinder: Schmidt, Joachim, Dr., 51427 Bergisch Gladbach (DE)

(57) **Zusammenfassung**

Die Erfindung betrifft ein Instrumentarium bestehend aus Ahle und Raspel zur Markraumvorbereitung bei der Implantation von Endoprothesen in Röhrenknochen. Erfindungsgemäß ist deren Schaff (2, 2a) als Hohlkörper (3, 3a) ausgebildet. An den an der Schaftaußenseite angebrachten Schneidzähnen (4, 4a) befinden sich in den Schaftinnenraum gerichtete Öffnungen (5, 5a).

Der Abraum sammelt sich in dem Hohlraum (3, 3a) des Ahlen- bzw. Raspelschaftes und kann von dort über Öffnungen am proximalen (6) und distalen (7) Ende des Schaftes durch Spülen und/oder Absaugen entfernt werden, oder wird nach Benutzung des Instrumentes mit diesem entfernt.

## Beschreibung

Die Erfindung betrifft ein Instrumentarium zur Markraumvorbereitung bei der Implantation von Endoprothesen in Röhrenknochen. Dieses Instrumentarium besteht aus einer Ahle und einer Raspel.

Bei jeder Implantation von Prothesen in den Markraum eines Röhrenknochens (hüftnaher Oberschenkelschaft, schulternaher Oberarmschaft, kniegelenksnaher Schienbein- und Oberschenkelschaft etc.) muß dieser Markraum für die Form und Größe der gewählten Prothese vorbereitet werden. Hierbei ist zu berücksichtigen, ob die Verankerung der Prothese mit oder ohne Zement erfolgen soll. Diese Vorbereitung geschieht in der Regel mit Ahlen und Raspeln, die auf die spätere Prothesenform abgestimmt sind. Hierzu ist nach Eröffnung des Knochens durch z.B. Entfernung des Gelenkkopfes Spongiosa und Mark auszuräumen und ein Lager zur Aufnahme des Prothesenschaftes zu schaffen. Dieser Raum muß so bemessen sein, daß die Prothese sich zementfrei verklemmt oder ausreichend Platz für eine zementfixierte Verankerung verbleibt und der Zement sich mit den verbleibenden Spongiosabälkchen verzahnt.

Bei gängigen Ahlen und Raspeln dieser Art, die in aller Regel aus einem metallischen Werkstoff gefertigt sind, entspricht deren Schaft in Dimensionierung und äußerer Formgebung exakt oder weitestgehend dem Schaft der einzusetzenden Prothese. Normalerweise besitzt die Ahle einen zylindrischen Schaft, während die Raspel den eigentlichen Prothesenschaft nachbildet. Die Außenfläche des Ahlen- bzw. Raspelschaftes ist mit scharfen Zähnen besetzt. Durch Einführen des Ahlenschaftes, in den Markraum, in der Regel mit Drehbewegung im Uhrzeigersinn, wird der Markraum eröffnet und achsengerecht vorbreitet. Durch Eindrücken des Raspelschaftes in den eröffneten Röhrenknochen, Auf- und Ab- bzw. Hin- und Herbewegung wird ein passendes Lager für die Prothese geschaffen.

Bei den bisherigen Ahlen und Raspeln verbleibt der Abraum, hauptsächlich abgespanter Knochen, in der Markhöhle. Der Abraum wird dort weiter zerrieben und vermischt sich mit Blut, Weichteilgewebe, eventueller Spülflüssigkeit etc. Dieses Abraumgemisch drückt sich durch die Raspelbewegungen im wesentlichen durch die gelenknahe Knocheneröffnung heraus und verteilt sich dort in den Weichteilen, sofern es nicht ausreichend aufgefangen und entfernt werden kann. Ein Teil drückt sich in die Spongiosawaben und ein weiterer Teil nach peripher in den Markraum. Um ein Einpressen des Abraumes in die Spongiosawaben zu vermeiden bzw. diese wieder zu eröffnen, muß während der Markraumvorbereitung das Instrument häufiger entfernt werden, um den Markraum zu spülen. Die Spülflüssigkeit verteilt sich ebenfalls in den gelenknahen Weichteilen.

Derartige Instrumente und ihre Handhabung sind von Nachteil:

Die Ausschwemmung von Knochenmaterial in die Weichteile wird u.a. für die Entstehung der heterotopen Ossifikationen verantwortlich gemacht, die z.B. in 30 %-80 % der Hüftendoprothetik das Ergebnis beeinträchtigen.

Die Spongiosawaben werden durch den Abraum verschlossen. Zement kann nicht zur Verankerung in diese Waben eindringen und sich damit auch nicht ausreichend verzahnen.

Abraum in den Spongiosawaben kann während der Bearbeitung bei der Einbringung des Zementes oder bei der Einbringung der Prothese in den Blutkreislauf eingepreßt werden und ein Fett-Embolie-Syndrom auslösen.

Während der Markraumvorbereitung muß das Instrumentarium deshalb häufig entfernt werden, um den Abraum aus der Markhöhle auszuspülen.

Aufgabe der vorliegenden Erfindung war es daher, ein Instrumentarium für die Markraumvorbereitung bei der Endoprothetik von Röhrenknochen zu schaffen, das die vorstehend beschriebenen Nachteile nicht aufweist.

Erfindungsgemäß wird dies durch ein Instrumentarium bestehend aus Ahle und Raspel für die Markraumvorbereitung gelöst, bei denen jeweils der Schaft als Hohlkörper ausgebildet ist und an deren Schneidzähne sich in den Schaftinnenraum gerichtete Öffnungen befinden. Durch diese konstruktiven Maßnahmen ist bei den erfindungsgemäßen Ahlen und Raspeln gewährleistet, daß bei deren Handhabung in an sich üblicher Weise abgespantes Knochenmaterial und Knochenmarkbestandteile in den Schaftinnenraum geführt werden Dort wird dieses während des Vorganges der Markraumvorbereitung gesammelt und dann in einem mit dem jeweiligen Instrument entfernt.

Vorteilhaft ist eine Absaugung des sich im Schaftinnenraum ansammelnden Abraumes. Zweckmäßigerweise ist hierzu am proximalen Ende des Instrumentes eine in den Innenraum reichende Öffnung vorgesehen, durch die eine Absaugeinrichtung geführt oder über eine Anschlußvorrichtung angeschlossen werden kann.

Die Abraumentfemung kann zusätzlich durch periodisches oder kontinuierliches Spülen gefördert werden. Hierzu ist es zweckmäßig, eine kombinierte Spül- und Absaugvorrichtung an der proximalen Öffnung des Ahlen- oder Raspelschaftes anzuschließen, durch die etwa mittels einer Kanüle Spülflüssigkeit in und durch den Instrumenteninnenraum geführt wird. Besonders vorteilhaft ist es, wenn das Instrument auch am distalen Ende eine Öffnung aufweist. Reicht das Ende der Spülkanüle bis dort hin oder durch diese in den Markraum hinaus, so wird durch die Spülflüssigkeit vornehmlich die Außenseite des Ahlen- oder Raspelkörpers umspült und der Abraum besonders effektiv durch die Öffnungen an den Schneidzähnen in den Schaftinnenraum geführt, von wo aus abgesaugt werden kann.

Im Falle der Ausführungsform der Instrumente mit zusätzlichen Öffnungen am proximalen und distalen Ende können diese auch in für die entsprechenden Prothesen an sich bekannter Weise über eine Zentriervorrichtung geführt werden. Als derartige Zentriervorrichtung kann ein Stab dienen, der an einen vorher in den Markraum gesetzten Markraumsperrer angebracht ist. Zentriervorrichtung und Spülkanüle können auch in Kombination vorliegen.

Die mit der Erfindung erzielbaren Vorteile sind:

Eine Einschwemmung von Knochenmarkbestandteilen in die gelenknahen Weichteile wird vermieden.

Ein Einpressen von Abraum in die Spongiosawaben wird vermieden.

Das Einpressen von Markraumbestandteilen in die Blutbahn (Fett-Embolie-Syndrom) wird reduziert, da einerseits kein Abraum aus den Spongiosawaben in die Blutbahn eingepreßt werden kann und andererseits die offene Konstruktion des Instrumentes einen pathologischen Druckaufbau vermeidet.

Eine bessere Aussteifung der Spongiosabälkchen bei der Zementierung wird erreicht, da die Spongiosawaben nicht durch Abraum verschlossen werden.

Der Operationsvorgang wird beschleunigt, da das bislang erforderliche zwischenzeitliche Spülen unterbleiben kann.

Zusätzlich wird bei dem erfindungsgemäßen Instrumentarium durch die offene Konstruktion im Gegensatz zu den sonst üblichen geschlossenen Vollkörpern ein intramedullärer Druckanstieg mit Auslösung eines Fett-Embolie-Syndroms vermieden.

Das erfindungsgemäße Instrumentarium zur Markraumvorbereitung kann bei allen Implantationen von Prothesen in Markräume (Oberschenkel, Oberarm, Schienbein etc.) bei der zementfixierten und zementfreien Prothesenverankerung genutzt werden.

Die Ahle bzw. Raspel wird in der der jeweiligen Prothese entsprechenden Form und Größe gefertigt und wie üblich benutzt. Bei kontinuierlicher Absaugung wird zusätzlich eine Saugvorrichtung an das Instrument angeschlossen. Bei zusätzlicher direkter Spülung ebenfalls ein Spülsystem. Während der Markraumvorbereitung kann ein zusätzliches Ausspülen des Markraumes vollständig unterbleiben oder auf ein Minimum reduziert werden. Eine Ausbreitung von Knochenmaterial in die Weichteile ist so zu vermeiden.

Die Erfindung wird nachfolgend am Beispiel einer Ahle und einer Raspel für eine Femurprothese näher beschrieben.

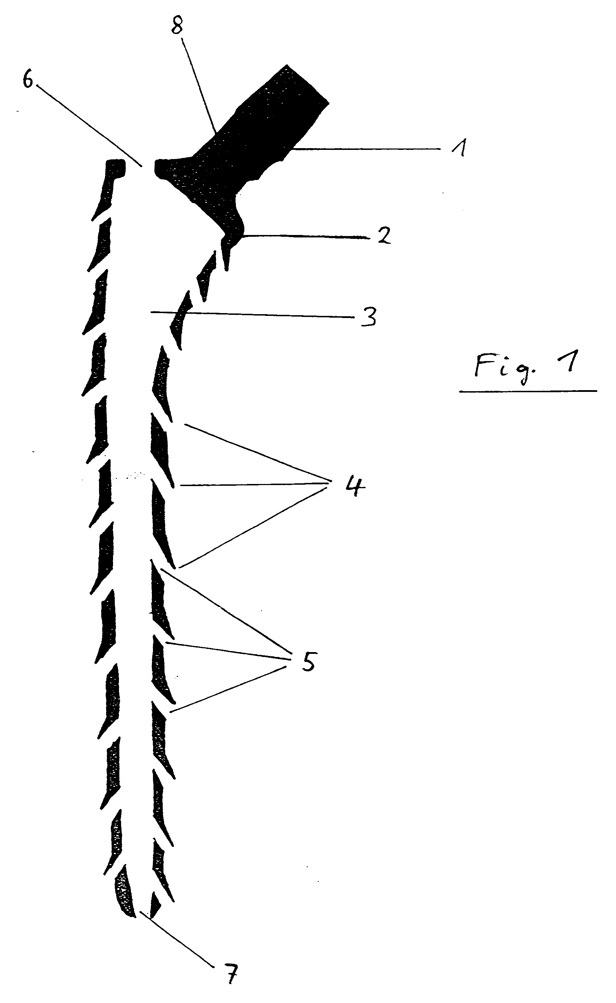

Fig. 1 zeigt als Ausführungsbeispiel eine Raspel im Längsschnitt. Die Raspel 1 entspricht in der äußeren Formgebung herkömmlichen Raspeln für Femurprothesen. Insbesondere ist der Raspelschaft dem Prothesenschaft nachgebildet. Mit diesem Instrument wird nach Gelenkkopfresektion und Markraumvorbereitung das Lager für den Prothesenschaft geschaffen.

Der Raspelschaft 2 ist als Hohlkörper 3 ausgebildet. Auf der Schaftaußenseite sind Schneidzähne 4 angebracht, die schräg nach unten gerichtet sein können. An den Innenflanken der Schneidzähne befinden sich Öffnungen, die entsprechend der Stellung der Schneidzähne in den Innenraum des als Hohlkörper 3 ausgebildeten Raspelschaftes gerichtet sind. Am proximalen und am distalen Ende des Raspelschaftes befinden sich je eine Öffnung 6 und 7. Durch diese kann gegebenenfalls eine Zentriervorrichtung (nicht gezeigt) geführt werden. Weiterhin kann an der proximalen Öffnung 6 eine Absaugeinrichtung (nicht gezeigt) oder eine kombinierte Spül-Absaugeinrichtung (nicht gezeigt) angeschlossen werden. Hals- und Konusbereich 8 der Raspel können in an sich bekannter Weise ausgestaltet und insbesondere zur Aufnahme eines Haltegriffes (nicht gezeigt) zur Führung der Raspel oder zur Aufnahme eines Probekopfes vorgesehen sein.

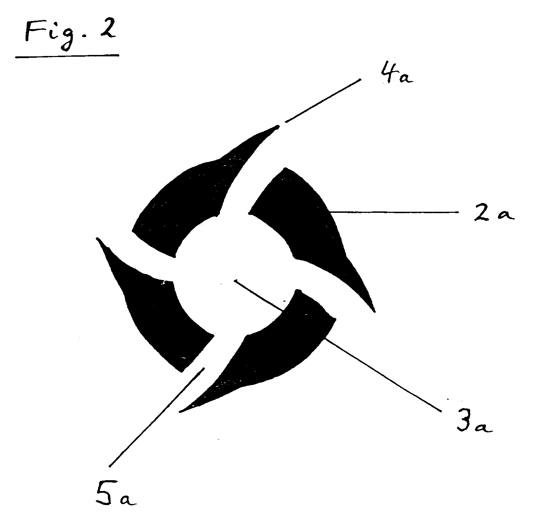

Fig. 2 zeigt eine Ahle im Querschnitt. Mit diesem Instrument wird nach der Gelenkkopfresektion der Markraum in der Weise vorbereitet, in dem es mittels Drehbewegung, hier im Uhrzeigersinn, in den Knochen eingeführt und dieser ausgeräumt wird. An dem als zylindrischer Hohlkörper 3a ausgebildeten Ahlenschaft 2a sitzen seitwärts nach außen gerichtete Schneidzähne 4a. Die Öffnungen 5a zum Instrumenteninnenraum 3a folgen der Richtung der Zahnstellung.

## Patentansprüche

1. Instrumentarium bestehend aus Ahle und Raspel zur Markraumvorbereitung bei der Endoprothetik von Röhrenknochen, bei denen jeweils der Schaft in Dimension und Formgebung dem Prothesenschaft entspricht und die Schaftaußenseite mit Schneidzähnen versehen ist, dadurch gekennzeichnet, daß der Raspelschaft (2, 2a) als Hohlkörper (3, 3a) ausgebildet ist und sich an den Schneidzähnen (4, 4a) in den Schaftinnenraum gerichtete Öffnungen (5, 5a) befinden.

2. Instrumentarium nach Anspruch 1, dadurch gekennzeichnet, daß sich die Öffnungen (5, 5a) an den Innenflanken der Schneidzähne (4, 4a) befinden.

3. Instrumentarium nach Anspruch 1 oder 2, dadurch gekennzeichnet, daß die Schneidzähne (4, 4a) schräg nach unten oder seitwärts nach außen gerichtet sind.

4. Instrumentarium nach einem der Ansprüche 1 bis 3, dadurch gekennzeichnet, daß es am proximalen Ende des Schaftes (2, 2a) eine Öffnung (6) aufweist, an die eine Absaugeinrichtung oder eine Spül-Absaugeinrichtung angeschlossen werden kann.

5. Instrumentarium nach Anspruch 4, dadurch gekennzeichnet, daß es am distalen Ende des Schaftes (2, 2a) eine Öffnung (7) aufweist.
